(19) Europäisches Patentamt
European Patent Office
Office européen des brevets

(11) Veröffentlichungsnummer: **0 028 813**
**B1**

(12) # EUROPÄISCHE PATENTSCHRIFT

(45) Veröffentlichungstag der Patentschrift: **25.07.84**

(51) Int. Cl.³: **A 61 K 9/00**, A 61 K 31/48

(21) Anmeldenummer: **80106843.8**

(22) Anmeldetag: **06.11.80**

(54) **Stabile Lösungen von hydrierten Ergotalkaloiden bzw. ihren Salzen und Heparin bzw. seinen Salzen sowie Verfahren zu deren Herstellung.**

(30) Priorität: **12.11.79 DE 2945636**

(43) Veröffentlichungstag der Anmeldung: **20.05.81 Patentblatt 81/20**

(45) Bekanntmachung des Hinweises auf die Patenterteilung: **25.07.84 Patentblatt 84/30**

(84) Benannte Vertragsstaaten: **DE FR IT NL SE**

(56) Entgegenhaltungen:
**DE-A- 2 555 481**
**DE-A- 2 625 403**
**DE-A- 2 735 587**
**DE-A- 2 945 677**
**GB-A- 2 015 338**

(73) Patentinhaber: **SANDOZ AG**
**Lichtstrasse 35**
**CH-4002 Basel (CH)**

(72) Erfinder: **Hartmann, Volker, Dr.**
**Stulbenweg 1**
**D-8500 Nürnberg (DE)**
Erfinder: **Otto, Karl-Heinz**
**Hohenburger Strasse 21**
**D-8455 Kastl/Opf. (DE)**
Erfinder: **Patt, Ludwig, Dr.**
**Ebenseestrasse 8**
**D-8500 Nürnberg (DE)**

**EP 0 028 813 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft eine stabile Lösung enthaltend als Wirkstoffe

a) Verbindungen der Formel I,

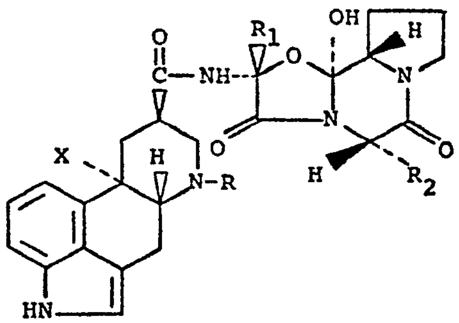

(I)

worin

R für Wasserstoff oder Alkyl mit 1-4 Kohlenstoffatomen,

$R_1$ für Methyl, Aethyl oder Isopropyl,

$R_2$ für Isopropyl, sek. Butyl, Isobutyl oder Benzyl, und

X für Wasserstoff oder Methoxy stehen,

bzw. deren pharm. annehmbare Salze und

b) Heparin bzw. dessen pharm. annehmbaren Salze.

Bei der Kombination von

a) Verbindungen der Formel I, insbesondere von Dihydroergotamin bzw. seinen pharm. annehmbaren Salzen wie dem Methansulfonat, Dihydroergovalin bzw. seinen pharm. annehmbaren Salzen wie dem Methansulfonat und 6-nor-6-Isopropyl-9,10-dihydro-2'β-methyl-5'α-benzyl-ergopeptin bzw. seinen pharm. annehmbaren Salzen wie dem Maleat, und

b) Heparin bzw. seinen pharm. annehmbaren Salzen, insbesondere dem Natriumsalz

in einer pharmazeutischen Zubereitung, war es bisher nicht möglich, klare und über einen längeren Zeitraum haltbare Lösungen herzustellen. Die beiden Komponenten sind in physiologisch verträglichen Lösungsmitteln bzw. deren Gemischen schlecht nebeneinander haltbar und reagieren unter Bildung eines schwer löslichen Salzes.

Diese Salzbildung kann einmal wie in der GB-OS-2 015 338 beschrieben durch die Verwendung von beispielsweise Dihydroergotamin-methansulfonat in Form einer festen Lösung mit Kollidon zeitweise verzögert, aber auf die Dauer nicht ganz unterdrückt werden, ein Befund, der zur Entwicklung des Lyophilisates als Arzneiform zur Injektion geführt hat. Zum anderen ist es auch möglich, durch die Auswahl eines geeigneten Lösungsmittelgemisches die Reaktion der beiden Wirkstoffe zu verzögern. Auf dieser Eigenschaft beruht die Entwicklung einer Zubereitung in Form einer Zweikammerspritze gemäss der DE-OS-2 945 677.

Diese enthält in getrennten Kompartimenten jeweils eine Lösung von Verbindungen der Formel I bzw. deren Salzen, insbesondere des Dihydroergotaminmethansulfonats und von Heparin bzw. seinen Salzen, insbesondere des Heparin-Natrium. Die Lösungen sind in der Spritze mischbar, und die Mischung ist über einige Stunden haltbar.

Es wurde nunmehr gefunden, dass man Lösungen von Verbindungen der Formel I bzw. deren pharm. annehmbaren Salzen und Heparin bzw. dessen pharm. annehmbaren Salzen in einem Verhältnis von 1 zu 500 vis 70 000, vorzugsweise von 1 zu 2 000 bis 20 000, jeweils bezogen auf mg der freien Base von Verbindungen der Formel I und I. E. der freien Säure des Heparins herstellen und die Lösungen über längere Zeiträume (gegen Ausfällung) stabil halten kann, falls man als stabilisierendes Medium ein Gemisch enthaltend

c) Wasser,

d) aliphatische Mono- und/oder Polyalkohole,

e) einen Stabilisator aus der Klasse der N-Acyl-Derivate des Anilins, und

f) Harnstoff und/oder Mono-Calcium-di-Natrium-Aethylendiamintetraacetat.

verwendet. Die so hergestellten Lösungen werden gegebenenfalls auf einen pH-Wert von 4 bis 6 eingestellt.

In dem erfindungsgemässen Medium verwendet man zusammen mit c) Wasser, das in einem Anteil von 45 bis 72 % eingesetzt wird, d) die aliphatischen Mono- und/oder Polyalkohole in einem Anteil von 28 bis 55 % (jeweils bezogen auf das Endvolumen des Lösungsmittelgemisches), wobei als Alkohole bzw. Polyalkohole Aethanol, Propylenglykol, Polyäthylenglykol (mittl. Molekulargewicht 400), Diäthylenglykol und Glyzerin verwendet werden. Die Kombinationen von Aethanol und Triäthylenglykol (Gewichtsverhältnis 1 zu 6 bis 10, insbesondere 8) und Glyzerin und Propylenglykol (Gewichtsverhältnis 1 zu 8 bis 12, insbesondere 10) werden hierbei bevorzugt. Als e) Stabilisatoren aus der Klasse der N-Acyl-Derivate des Anilins verwendet man vorzugsweise 2-(Diäthylamino)-N-(2,6-dimethylphenyl) acetamid, 2-(Butylamino)-N-(2-chlor-6-methylphenyl)-acetamid und 2-(2-Diäthylaminoacetamido)-m-toluolsäuremethylester bzw.

deren Salze, beispielsweise Hydrochloride. Sie sollen sich in einer Konzentration von 1 bis 2 % bezogen auf die Gesamtmenge der fertigen Zusammensetzung befinden. f) Harnstoff bzw. das Mono-Calcium-di-Natrium-Aethylendiamin-tetraacetat sollen bezogen auf 5 000 I. E. Heparin in einem Anteil von 2-5 mg in dem Gemisch anwesend sein.

Das Verfahren wird vorzugsweise so durchgeführt, dass man zunächst unter Schutzbegasung, insbesondere $CO_2$-Begasung, eine Lösung von a) Verbindungen der Formel I bzw. deren pharm. annehmbaren Salzen, insbesondere Dihydroergotamin bzw. dessen Salzen, insbesondere des Methansulfonates (-mesilates) in aliphatischen Mono- und/oder Polyalkoholen, d. i. einer Komponente gemäss d), beispielsweise dem Gemisch von Glyzerin und Propylenglykol, in Gegenwart eines Stabilisators aus der Klasse der N-Acyl-Derivate des Anilins, d. i. einer Komponente gemäss e) herstellt und dieser Lösung anschliessend ebenfalls unter Schutzbegasung, insbesondere $CO_2$-Begasung, eine Lösung eines b) Heparin-Salzes, insbesondere des Natriumsalzes, in Wasser, d. i. einer Komponente gemäss c), in Anwesenheit von Harnstoff und/oder Mono-Calcium-di-Natrium-Aethylendiamin-tetraacetat (im Handel erhältlich unter dem Namen Calciumtitriplex · 6 $H_2O$), d. i. einer Komponente gemäss f), zusetzt.

Die Einstellung des pH-Wertes des so erhaltenen stabilisierten Mediums auf 4 bis 6 erfolgt zweckmässigerweise durch Zugabe von organischen Säuren, insbesondere der Methansulfonsäure.

Den erhaltenen Lösungen können noch Konservierungsmittel, beispielsweise Chlorkresol oder Trichlor-tert.-butanol in einem Anteil von 0,2 bis 1 % bezogen auf die endgültige Zusammensetzung zugesetzt werden. Die erhaltenen Lösungen werden zweckmässigerweise nach Sterilfiltration unter aseptischen Bedingungen, vorzugsweise unter Inertbegasung, in Ampullen oder Einmalspritzen abgefüllt. In dieser Form sind die stabilen Lösungen von Verbindungen der Formel I bzw. deren Salzen, wie den Methansulfonaten, Maleaten oder Tartraten, und Heparin bzw. seinen Salzen, wie den Natrium-, Kalium- oder Calciumsalzen, über längere Zeiträume, d. i. 2 bis 5 Jahre, haltbar. Die Verabreichung der so erhaltenen stabilen Lösungen erfolgt durch subcutane Injektion, wie beschrieben in der DOS-2 554 533.

## Beispiel 1

Herstellung einer Heparin- (5 000 IE) Dihydroergotamin- (0,5 mg) Lösung zur Injektion

a) Herstellung der Dihydroergotamin-Lösung

In eine 50 Liter Rührapparatur werden 18,4 kg Propylenglykol und 1,84 kg Glyzerin wasserfrei gegeben und das Gemisch unter $CO_2$-Begasung während 10 Minuten gerührt. Unter weiterem Rühren und $CO_2$-Begasung während ca. 30 Minuten werden dem Gemisch 0,028 6 kg Dihydroergotamin-mesilat und 0,426 kg Lidocain-hydrochlorid zugegeben und darin gelöst.

b) Herstellung der Heparin-Lösung

In einer 30 Liter Rührapparatur werden 18,4 kg Wasser (für Injektionszwecke) gegeben und 10 Minuten unter $CO_2$-Begasung gerührt. Unter weiterem Rühren und $CO_2$-Begasung werden während ca. 30 Minuten 1,898 kg Heparin-Natrium (entsprechend 285,7 Mio. IE) und 0,114 kg Mono-Calcium-di-Natrium-Aethylendiamin-tetraacetat (im Handel erhältlich unter dem Namen Calciumtitriplex · 6 $H_2O$) zugegeben und darin gelöst.

c) Mischung der unter a) und b) hergestellten Lösungen

Die gemäss b) hergestellte Lösung wird unter $CO_2$-Begasung der gemäss a) hergestellten Lösung zugesetzt. Danach wird das Gefäss, in dem die Lösung b) hergestellt wurde, mit 1 kg Wasser (für Injektionszwecke) ausgespült und dieses ebenfalls der gemäss a) hergestellten Lösung zugefügt. Die vereinigten Lösungen werden während 10 Minuten unter $CO_2$-Begasung gerührt (der pH-Wert der vereinigten Lösungen soll hierbei ca. 5,7 betragen) und danach durch Zugabe von Wasser (für Injektionszwecke) auf ein Endgewicht von 42,810 kg (entspricht 40 Ltr. Flüssigkeit) gebracht.

d) Filtration

$d_1$) Vorfiltration : Die Vorfiltration erfolgt durch ein Membranfilter (0,2 µm-Ultipor nm Pall).

$d_2$) Sterilfiltration : Die gemäss c) hergestellte und vorfiltrierte Lösung wird direkt an der Abfüllmaschine mit $CO_2$ über ein sterilisiertes Druckfiltrationsgerät mit Membranfilter (0,2 µm-Ultipor nm Pall) unter 1,7 bar Druck filtriert.

Die sterilfiltrierte Lösung wird unter aseptischen Bedingungen in 1 ml Ampullen abgefüllt (Füllvolumen 0,8 ml).

## Beispiel 2

Herstellung einer Heparin- (2 500 IE) Dihydroergotamin- (0,5 mg) Lösung zur Injektion

a) Herstellung der Dihydroergotamin-Lösung

Die Lösung wird unter Verwendung des in Beispiel 1a) beschriebenen Verfahrens mit 15,3 kg Propylenglykol, 1,53 kg Glyzerin, 0,03 kg Dihydroergotamin-mesilat und 0,32 kg Lidocainhydrochlorid hergestellt.

b) Herstellung der Heparin-Lösung

Die Lösung wird unter Verwendung des in Beispiel 1b) beschriebenen Verfahrens mit 0,997 kg Heparin-Natrium (entsprechend 150 Mio. IE) und 0,12 kg Mono-Calcium-di-Natrium-Aethylendiamintetraacetat (Calciumtitriplex $6.H_2O$) hergestellt.

c) Mischung der unter a) und b) hergestellten Lösungen

Die gemäss a) und b) hergestellten Lösungen werden vereinigt, die vereinigte Lösung unter $CO_2$-Begasung während 10 Minuten gerührt (pH-Wert soll ca. 5,7 betragen) und mit Wasser für Injektionszwecke auf ein Endgewicht von 32,04 kg aufgefüllt.

d) Filtration

$d_1$) Vorfiltration : Membranfilter 0,2 μm (Ultipor nm Pall).

$d_2$) Sterilfiltration : Die Lösung wird direkt an der Abfüllmaschine mit $CO_2$ über eine sterilisiertes Druckfiltrationsgerät mit Membranfilter 0,2 μm (Ultipor nm Pall) unter 1,7 bar Druck filtriert.

Die sterilfiltrierte Lösung wird unter aseptischen Bedingungen in 1 ml Ampullen abgefüllt.

**Ansprüche**

1. Eine stabile Lösung enthaltend als Wirkstoffe

a) Verbindungen der Formel I,

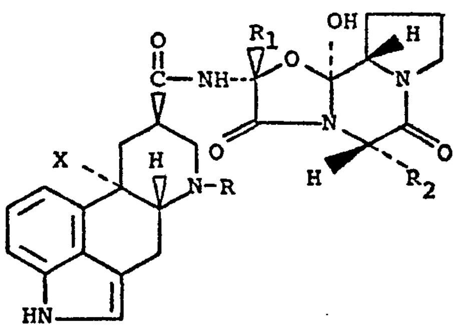

(I)

worin

R für Wasserstoff oder Alkyl mit 1-4 Kohlenstoffatomen,

$R_1$ für Methyl, Aethyl oder Isopropyl,

$R_2$ für Isopropyl, sec.-Butyl, Isobutyl oder Benzyl, und

X für Wasserstoff oder Methoxy stehen,

bzw. deren pharm. annehmbare Salze und

b) Heparin bzw. dessen pharm. annehmbare Salze

und als stabilisierendes Medium ein Gemisch von

c) Wasser,

d) aliphatischen Mono- und/oder Polyalkoholen,

e) einem Stabilisator aus der Klasse der N-Acyl-Derivate des Anilins und

f) Harnstoff und/oder Mono-Calcium-di-Natrium-Aethylendiamintetraacetat.

2. Eine stabile Lösung enthaltend als Wirkstoffe

a) Dihydroergotamin bzw. dessen pharm. annehmbare Salze und

b) Heparin bzw. dessen pharm. annehmbare Salze

und als stabilisierendes Medium ein Gemisch von

c) Wasser,

d) aliphatischen Mono- und/oder Polyalkoholen,

e) 2-(Diethylamino)-N-(2,6-dimethylphenyl)acetamid, 2-Butylamino)-N-(2-chlor-6-methylphenyl)-acetamid, 2-(2-Diethylamino-acetamido)-m-toluolsäuremethylester bzw. deren Salze und

f) Harnstoff und/oder Mono-Calcium-di-Natrium-Aethylendiamintetraacetat.

3. Eine Lösung gemäss den Ansprüchen 1 und 2, die einen pH-Wert von 4-6 besitzt.

4. Eine Lösung gemäss den Ansprüchen 1 und 2, die als Verbindungen der Formel I bzw. deren pharm. annehmbare Salze Dihydroergotamin, Dihydroergovalin oder 6-nor-6-Isopropyl-9,10-dihydro-2'β-methyl-5'α-benzylergopeptin bzw. deren pharm. annehmbare Salze enthält.

5. Eine Lösung gemäss Anspruch 3, die Dihydroergotamin und Dihydroergovalin als Methansulfonate und 6-nor-6-Isopropyl-9,10-dihydro-2'β-methyl-5'α-benzyl-ergopeptin als Maleat enthält.

6. Eine Lösung gemäss den Ansprüchen 1 und 2, die Heparin in Form des Natriumsalzes enthalt.

7. Eine Lösung gemäss den Ansprüchen 1, 2 und 4, worin sich eine Verbindung der Formel I bzw. deren pharm. annehmbare Salze und Heparin bzw. dessen pharm. annehmbare Salze in einem Verhältnis von 1 zu 500 bis 70 000 — jeweils bezogen auf mg der freien Base von Verbindungen der Formel I und I.E. der freien Säure des Heparins — befindet.

8. Eine Lösung gemäss Anspruch 7, worin sich eine Verbindung der Formel I bzw. deren pharm. annehmbare Salze und Heparin bzw. dessen pharm. annehmbare Salze in einem Verhältnis von 1 zu 2 000 bis 20 000 — jeweils bezogen auf mg der freien Base von Verbindungen der Formel I und I.E. der freien Säure des Heparins — befindet.

9. Eine Lösung gemäss den Ansprüchen 1 und 2, worin sich die Komponente c) in einem Anteil von 45 bis 72 % bezogen auf das Gesamtvolumen der Lösung befindet.

10. Eine Lösung gemäss den Ansprüchen 1 und 2, enthaltend als Komponente d) Aethanol, Propylenglykol mit einem Molekulargewicht von ca. 400, Diäthylenglykol und Glyzerin.

11. Eine Lösung gemäss Anspruch 10, worin sich die Komponente d) in einem Anteil von 28 bis 55 % bezogen auf das Gesamtvolumen der Lösung befindet.

12. Verfahren zur Herstellung der Lösung gemäss den Ansprüchen 1, 2 und 4, dadurch gekennzeichnet, dass man jeweils unter Schutzbegasung

a) Verbindungen der Formel I

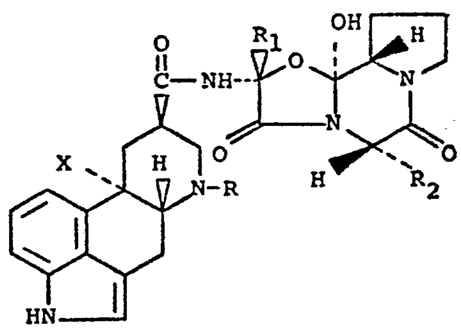

(I)

worin

R für Wasserstoff oder Alkyl mit 1-4 Kohlenstoffatomen,

$R_1$ für Methyl, Aethyl oder Isopropyl,

$R_2$ für Isopropyl, sec.-Butyl, Isobutyl oder Benzyl und

X für Wasserstoff oder Methoxy stehen,

bzw. deren pharm. annehmbare Salze in einer Komponente gemäss d) in Gegenwart einer Komponente gemäss e) löst und dieser Lösung ebenfalls unter Schutzbegasung eine Lösung von

b) Heparin bzw. dessen pharm. annehmbaren Salzen in einer Komponente gemäss c) in Anwesenheit einer Komponente gemäss f) zusetzt und die Lösung gegebenenfalls auf einen pH-Wert von 4-6 einstellt.

## Claims

1. A stable solution containing as active agents

a) compounds of formula I

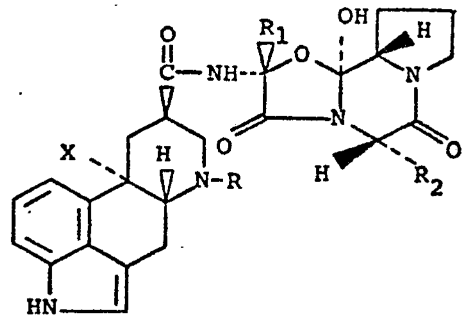

(I)

wherein

R represents hydrogen or alkyl with 1-4 carbon atoms,

$R_1$ represents methyl, ethyl or isopropyl,

$R_2$ represents isopropyl, sec.-butyl, isobutyl or benzyl, and

X represents hydrogen or methoxy,

or their pharmaceutically acceptable salts and

b) heparin or its pharmaceutically acceptable salts and as stabilizing medium a mixture of

c) water,

d) aliphatic mono- and/or polyalcohols,

e) a stabilizor from the class of N-acyl-derivatives of aniline and

f) urea and/or mono-calcium-di-sodium-ethylenediaminetetraacetate.

2. A stable solution containing as active agents

a) dihydroergotamine or its pharmaceutically acceptable salts and

b) heparin or its pharmaceutically acceptable salts and as stabilizing medium a mixture of

c) water,

d) aliphatic mono- and/or polyalcohols,

e) 2-(diethylamino)-N-(2,6-dimethylphenyl)-acetamide, 2-(butylamino)-N-(2-chloro-6-methylphenyl)-acetamide, 2-(2-diethylamino-acetamido)-m-toluic acid methyl ester or their salts and

f) urea and/or mono-calcium-di-sodium-ethylenediaminetetraacetate.

3. A solution according to claims 1 and 2, having a pH-value of 4-6.

4. A solution according to claims 1 and 2, containing dihydroergotamine, dihydroergovaline or 6-nor-6-isopropyl-9,10-dihydro-2'β-methyl-5'α-benzylergopeptine or their pharmaceutically acceptable salts as compounds of formula I or their pharmaceutically acceptable salts.

5. A solution according to claim 3, containing dihydroergotamine and dihydroergovaline as the methanesulfonates and 6-nor-6-isopropyl-9,10-dihydro-2'β-methyl-5'α-benzyl-ergopeptine as the maleate.

6. A solution according to claims 1 or 2, containing heparin in the form of the sodium salt.

7. A solution according to claims 1, 2 and 4, wherein a compound of formula I or its pharmaceutically acceptable salts and heparin or its pharmaceutically acceptable salts is present in a ratio of from 1 to 500 to 70 000 — based in each case on mg of the free base of the compound of formula I and I.U. of the free acid of heparin.

8. A solution according to claim 7, wherein a compound of formula I or its pharmaceutically acceptable salts and heparin or its pharmaceutically acceptable salts is present in a ratio of from 1 to 2 000 to 20 000 — based in each case of mg of the free case of the compound of formula I and I.U. of the free acid of heparin.

9. A solution according to claims 1 and 2, wherein component c) is present in a amount of 42 to 72 % based on the total volume of the solution.

10. A solution according to claims 1 and 2, containing ethanol, propylene glycol with a molecular weight of ca. 400, diethyleneglycol and glycerine as component d).

11. A solution according to claim 10, wherein component d) is present in an amount of from 28 to 55 % based on the total volume of the solution.

12. Process for the production of the solution according to claims 1, 2 and 4, characterized in that

a) compounds of formula I

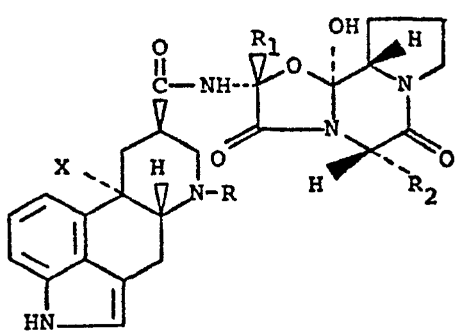

(I)

wherein

R represents hydrogen or alkyl with 1-4 carbon atoms,

$R_1$ represents methyl, ethyl or isopropyl,

$R_2$ represents isopropyl, sec.-butyl, isobutyl or benzyl and

X represents hydrogen or methoxy

or their pharmaceutically acceptable salts, is dissolved in a component in accordance with d) in the presence of a component according to e) in each case under protective gassing and to this solution is added a solution of b) heparin or its pharmaceutically acceptable salts in a component according to c) in

the presence of a component according to f) also under protective gassing, and the solution is optionally adjusted to a pH-value of 4-6.

**Revendications**

1. Une solution stable contenant comme principes actifs
a) des composés de formule I

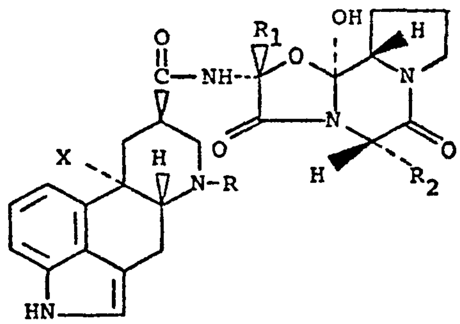

(I)

dans laquelle
R représente l'hydrogène ou alkyle contenant de 1 à 4 atomes de carbone,
$R_1$ représente méthyle, éthyle ou isopropyle,
$R_2$ représente isopropyle, sec.-butyle, isobutyle ou benzyle, et
X représente l'hydrogène ou méthoxy,
ou leurs sels pharmaceutiquement acceptables et
b) de l'héparine ou ses sels pharmaceutiquement acceptables et comme milieu stabilisant un mélange
c) d'eau,
d) de mono- et/ou de polyalcools aliphatiques,
e) d'un stabilisant de la classe des dérivés N-acylés de l'aniline et
f) d'urée et/ou d'éthylènediaminetétraacétate monocalcique-di-sodique.

2. Une solution stable contenant comme principes actifs
a) de la dihydroergotamine ou ses sels pharmaceutiquement acceptables et
b) de l'héparine ou ses sels pharmaceutiquement acceptables et comme milieu stabilisant un mélange
c) d'eau,
d) de mono- et/ou de polyalcools aliphatiques,
e) de 2-(diéthylamino)-N-(2,6-diémthylphényl) acétamide, de 2-(butylamino)-N-(2-chloro-6-méthyl-phényl)-acétamide, d'ester méthylique de l'acide 2-(2-diéthylamino-acétamido)-m-toluique ou leurs sels et
f) d'urée et/ou d'éthylènediaminetétraacétate monocalcique-disodique.

3. Une solution selon les revendications 1 et 2, qui possède une valeur de pH de 4-6.

4. Une solution selon les revendications 1 et 2, qui contient comme composés de formule I ou leurs sels pharmaceutiquement acceptables, de la dihydroergotamine, de la dihydroergovaline ou de la 6-nor-6-isopropyl-9,10-dihydro-2'β-méthyl-5'α-benzylergopeptine ou leurs sels pharmaceutiquement acceptables.

5. Une solution selon la revendication 3, qui contient de la dihydroergotamine et de la dihydroergovaline à l'état de méthanesulfonates et de la 6-nor-6-isopropyl-9,10-dihydro-2'β-méthyl-5'α-benzyl-ergopeptine à l'état de maléate.

6. Une solution selon les revendications 1 et 2, qui contient l'héparine sous forme de sel de sodium.

7. Une solution selon les revendications 1, 2 et 4, dans laquelle un composés de formule I ou ses sels pharmaceutiquement acceptables et de l'héparine ou ses sels pharmaceutiquement acceptables sont présents dans un rapport de 1 à 500 jusqu'à 70 000 — rapporté dans chaque cas au mg de la base libre des composés de formule I et aux U.I. de l'acide libre de l'héparine.

8. Une solution selon la revendication 7, dans laquelle un composé de formule I ou ses sels pharmaceutiquement acceptables et l'héparine ou ses sels pharmaceutiquement acceptables sont présents dans un rapport de 1 à 2 000 jusqu'à 20 000 — rapporté dans chaque cas au mg de la base libre des composés de formule I et aux U.I. de l'acide libre de l'héparine.

9. Une solution selon les revendications 1 et 2, dans laquelle le composant c) est présent en une proportion de 45 à 72 % rapportée au volume total de la solution.

10. Une solution selon les revendications 1 et 2, contenant comme composant d) de l'éthanol, du propylèneglycol ayant un poids moléculaire d'environ 400, du diéthylèneglycol et de la glycérine.

11. Une solution selon la revendication 10, dans laquelle le composant d) est présent en une proportion de 28 à 55 % rapportée au volume total de la solution.

12. Procédé de préparation de la solution selon les revendications 1, 2 et 4, caractérisé en ce que, chaque fois sous protection de gaz, on dissout

a) des composés de formule I

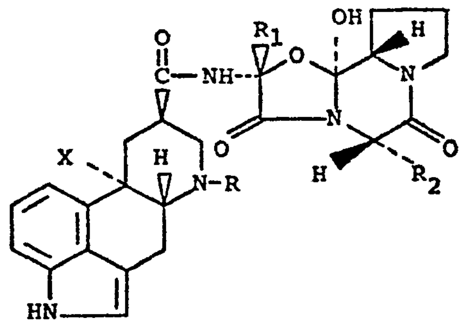

(I)

dans laquelle

R représente l'hydrogène ou alkyle contenant de 1 à 4 atomes de carbone,

$R_1$ représente méthyle, éthyle ou isopropyle,

$R_2$ représente isopropyle, sec.-butyle, isobutyle ou benzyle et

X représente l'hydrogène ou méthoxy,

ou leurs sels pharmaceutiquement acceptables, dans un composant selon d) en présence d'un composant selon e) et on ajoute à cette solution, également sous protection de gaz, une solution

b) d'héparine ou de ses sels pharmaceutiquement acceptables dans un composant selon c) en présence d'un composant selon f) et on règle éventuellement la solution à une valeur de pH de 4-6.